# EUROPEAN PATENT SPECIFICATION

(11) **EP 1 656 961 B1**
(45) Date of publication and mention of the grant of the patent: **05.08.2015**
(21) Application number: 05024079.5
(22) Date of filing: 31.12.2001
(51) Int. Cl.: A61L 31/08, A61L 31/14, A61L 31/16, A61L 33/00

(54) **Immuno-tolerant stent with surface microstructure**
Immunotoleranter Stent mit Oberflächen-Mikrostruktur
Stent immunotolérant ayant une surface microstructurée

(30) Priority: 02.05.2001 US 847626; 12.10.2001 EP 01124528
(43) Date of publication of application: 17.05.2006
(62) Divisional of application: 01131051.3
(73) Proprietor: InFlow Dynamics, Inc., Springfield, Virginia 22151 (US)
(72) Inventor: Alt, Eckhard, Dr., 81679 Munchen (DE)
(74) Representative: Peterreins, Frank

(56) References cited:
- EP-A- 0 806 212
- WO-A-00/10623
- DE-A- 19 916 086
- US-A- 5 735 896

## Description

The invention refers to a method of making a stent and a stent producible with said method.

Stents are widely used to support a lumen in the human body, especially the lumen of a coronary artery.

The problem of coronary restenosis has been one of the major challenges for modern interventional cardiology.

Despite major progress in stent design, stent technology, flexibility, delivery systems and other means to improve the technique of coronary stenting, coronary restenosis still is of concern. Roughly 25% of all patients which receive an intervention in their vascular bed receive an implant and suffer a restenosis, which means that more than 50% of the initial lumen gain are lost in the follow-up between 6 weeks and 6 months.

A wide range of solutions have been proposed including a modification of the stent surface by iridiumoxide or the coating of the stent surface with biodegradable or non-biodegradable coatings. It has been proposed to provide the stent surface with a coating of an immuno suppressing drug, namely "Tacrolimus", which prevents or at least reduces the immuno-response by a modification of the immuno-response of the body. These drugs are otherwise used to suppress the rejection of transplants in the human body. In addition, recent research has shown, that a rough stent surface may be advantageous to prevent restenosis. However, precise predictions and results could not be achieved as yet.

WO 00/10623 discloses a stent with a surface pattern comprising a plurality of depressions with an average diameter of about 30 - 65 micrometers to achieve an organised growth pattern of infiltrating cells in order to avoid immuno-reactions of the body leading to restenosis.

It is an object of the present invention to propose a method of making an alternative stent design, which provokes a minimum immuno-reaction by the body against the implanted foreign body, thus avoiding restenosis after implantation.

This object is solved by the features of claim 1.

Further embodiments and advantages of the invention are disclosed in the subclaims.

The solution is, to minimize the surface recognition of the stent as an implanted foreign body creating a stent surface, which is more immuno tolerant than a smooth or generally rough surface. It has shown, that the stent restenosis primarily is a foreign body recognition with concomitant inflammatory reaction against the implanted foreign body, very similar to the rejection of a transplanted organ with a foreign body surface to the body. This reaction primarily is brought forward by T-Lymphocytes. These Lymphocytes attack anything which is determined to be foreign and a release of Cytokines is affected following this recognition.

It could be shown, that the reaction to a transplanted foreign body is minimized, if a certain stent surface structure can be achieved which mimics the surface structure of the patient's own cells. By applying a surface structure of the stent having microstructures with lateral dimensions in the range of o.5 to 5 micrometers (µm) and especially 1 to 3 µm roughness, the body is blinded towards the foreign body and by surface characterization and immuno tolerance is induced.

This is absolutely in contrary to the current opinion that a stent surface should be smooth in order to be well tolerated by the human body. The experiment, that has been conducted at applicants research lab, has shown, that especially a structure with an inert material, such as iridiumoxide, niobiumoxide, titaniumnitrate or other very inert ceramic-like structures, suppress this immuno-reaction.

In addition, small traces of the immuno modulating drug "Tacrolimus" or Sirolimus" enhance the action towards a suppressed immuno reaction.

The consequences of this blinding of the immuno response by especially two factors, surface modification and in addition the adding of small traces of an immuno suppressing drug without any systemic effect, promote the healing of the stent without suppressing the proliferation of smooth muscle cells.

A problem of all currently proposed concepts with drugs was the assumption that only the inhibition of the proliferation would be sufficient to suppress restenosis. In contrary thereto, the aim of this invention is to allow the proliferation of smooth muscle cells to the same degree as they normally would proliferate, but suppress the continuous overshooting proliferation that goes beyond the wound healing. This overshooting proliferation is responsible for the restenosis and is based on an inflammatory response toward the foreign body which is recognized by the T-cell system.

The combined approach of blinding the recognition of the implanted foreign body by grading a structure in a range of 1 to 5µm, especially 1 to 3µm, by additionally using material that has very inert surface properties, has no release of toxic substances, has a positive surface charge in the range 3x10⁻² to 5x10⁻² N/m (30 to 50 dynes/cm (g x cm x s⁻²/cm)), which reduces fibrinogen adhesion, that also diminishes the inflammatory reaction towards the release of metallic salts from otherwise stainless steel, which is especially avoided by an inert ceramic-like structure, all these factors contribute to the diminished immuno response.

The additional application of an immuno suppressive drug such as Sirolimus or Tacrolimus helps to overcome the very weak inflammatory response toward such an implanted foreign body, which is not only due to the surface recognition, but also to the mechanical trauma which is brought forward by the implantation of the stent.

The application of the immuno drug can be effected either by dipping the stent in a solution of the drug. For example, 40 mg Tacrolimus are dissolved in 6 ml of Chloroform or Ethylacetate and on the stent surface soaked by adhesion forces and by capillary forces the drug into the surface coating. The stent is taken out of the solution and the solvent evaporates following the high pressure of the dissolved chloroform into the ambient atmosphere rapidly. The consequence is, that dependent on the initial concentration small traces of the drug are bound to the surface and soaked into the network, the grooves and the capillaries of the rough surface structure of the stent.

Thereby, the surface modification of the stent serves two purposes:
1. It blinds the recognition of the stent surface as a foreign body and
2. it facilitates and helps to retain traces in a range of 5 - 50 µg of immuno drug on the surface structure of the stent.

In addition the application of the drug by means of a biodegradable or non-biodegradable carrier can be done as well. So drugs had been described previously and are consisting of either biodegradable such as polylactic acid or non-biodegrable or non-erodable polymers such as polyurethane, polyvinylacetate or other.

Further details and advantages of the present invention are disclosed in the following description in connection with the annexed drawings, in which.
Fig. 1 is a perspective partial view of a stent made according to the invention;
Fig. 2 is a schematic view of a part D of the surface of the stent; and
Fig. 3 is a section according to line III - III in Fig. 2.

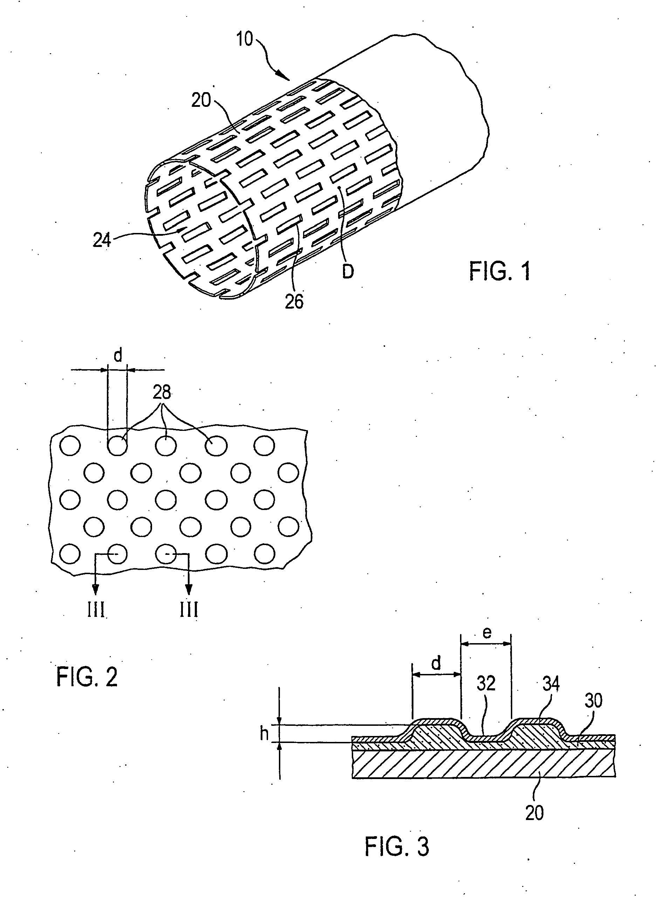

Fig. 1 shows a part of a stent 10 in form of a small tube 20 preferably made of a niob-zirconium alloy which has two open ends (one is shown at 24) and a lot of openings 26 in the wall of the tube 20, so that the stent may be crimped on an angioplasty balloon(not shown) and deployed by the balloon on a treatment site for example in a coronary artery from its shown first small diameter to a second greater diameter so that the outer surface of the stent is in contact with the inner wall surface of the artery. Many designs of the stent are possible and well known in the state of the art; for the invention the configuration of the stent is not relevant.

The outer surface of the stent 10 is modified and provided with small protrusions or microstructures 28 as shown in Figs. 2 and 3 for a small region D depicted in Fig. 1. These microstructures have a lateral extension d in the range of 0.5 to 5 micrometers, preferably in the range of 1 to 3 micrometers. The spacing e between adjacent microstructures is in the same range of 0.5 to 5 micrometers, preferably in the range of 1 to 3 micrometers, as schematically shown in **Fig. 3****.** The height **h** of the microstructures between the peak of a microstructure and the valley adjacent to a next microstructure may have a lower value and lie in the range of 0.5 to 2 micrometers.

The microstructures **28.** may be a coating **30** of a ceramic-like material such as iridiumoxide, which may be deposited on the surface of the surface of the stent by means of plasma or chemical vapour deposition or the like.

The microstructures **28** may have the form of microspheres or parts thereof; the surface of the microstructures may not be smooth and be provided with grooves or furrows **32** depending on the manufacturing process.

The stent is then dipped into a solution of an immuno suppressing drug, for example "Tacrolimus" or "Sirolimus", and a solvent such as chloroform, so that the solution covers the surface of the stent, partially by capillary forces in the region of the grooves **32**. The stent is then more or less covered by a coating **34** of the immuno suppressing drug.

## Claims

1. A method of making a stent for implantation in a lumen of the human body, comprising:
providing a stent that is provided with a rough outer surface, **characterised in that** the surface of the stent comprises microstructures having lateral extensions (d) in the range of 0.5 to 5 micrometers and separated from each other with spacings (e), whereby the recognition of the stent as an implanted foreign body is reduced; and
applying an immuno suppressing drug to the rough outer surface, whereby the immuno suppressing drug treats an inner surface of the lumen and prevents an immuno reaction thereof.

2. The method according to claim 1, **characterised in that** the applying step comprises dipping the stent in a solution comprising the immuno suppressing drug and a solvent.

3. The method according to claim 2, **characterised in that** the solution is applied to the surface of the stent by a capillary force of said surface.

4. The method according to claim 3, **characterised in that** the immuno suppressing drug adheres on the surface of the stent following the capillary force without using a carrier.

5. The method according to claim 4, **characterised in that** the immuno suppressing drug is soaked into the network, the grooves and the capillaries of the microstructures of the surface of the stent.

6. The method according to any one of the preceding claims, **characterised in that** the lateral extensions (d) of the microstructures are in the range of 1 to 3 micrometers.

7. The method according to any one of the preceding claims, **characterised in that** the spacings (e) between adjacent microstructures are in the range of 0.5 to 5 micrometers.

8. The method according to claim 7, **characterised in that** the spacings (e) between adjacent microstructures are in the range of 1 to 3 micrometers.

9. The method according to any one of the preceding claims, **characterised in that** the surface structure of the stent is inert and releases no toxic substances.

10. The method according to any one of the preceding claims, **characterised in that** the surface structure of the stent has a surface charge in the range of 3x10⁻² to 5x10⁻² N/m (30 - 50 dynes/cm (g x cm x s⁻² /cm)).

11. The method according to any one of the preceding claims, **characterised in that** the immuno suppressing drug is able to suppress an immuno reaction or response interacting with the T- cells of the human body.

12. The method according to any one of the preceding claims, **characterised in that** the immuno suppressing drug is sirolimus.

13. The method according to any one of the preceding claims, **characterised in that** the immuno suppressing drug is tacrolimus.

14. The method according to any one of the preceding claims, **characterised in that** the surface of the stent modified by the microstructures is made of iridium oxide.

15. The method according to any one of the preceding claims, **characterised in that** the surface of the stent modified by the microstructures is made of niobium oxide.

16. The method according to any one of the preceding claims, **characterised in that** the surface of the stent modified by the microstructures is made of titanium nitrate.

17. The method according to claim 13, **characterized in that** the solvent is chloroform.

18. The method according to claim 17, further **characterised in that** the chloroform is evaporated from the surface of the stent.

19. A stent for implantation in a lumen of the human body, said stent being producible with the method of any of claims 1 to 18.

## Patentansprüche

1. Ein Verfahren zur Herstellung eines Stents zur Implantierung in ein Lumen des menschlichen Körpers, umfassend:
Bereitstellen eines Stents, der versehen ist mit einer rauen äußeren Oberfläche, **dadurch gekennzeichnet dass** die Oberfläche des Stents Mikrostrukturen umfasst, die laterale Abmessungen (d) in dem Bereich von 0,5 bis 5 Mikrometern aufweisen und voneinander durch Abstände (e) getrennt sind,
wodurch die Erkennung des Stents als ein implantierter Fremdkörper verringert ist; und
Aufbringen eines immunsupprimierenden Arzneistoffs auf die raue äußere Oberfläche, wobei der immunsupprimierende Arzneistoff eine innere Oberfläche des Lumens behandelt und dessen Immunreaktion verhindert.

2. Verfahren nach Anspruch 1, wobei der Aufbringungsschritt das Eintauchen des Stents in eine Lösung umfassend den immunsupprimierenden Arzneistoff und ein Lösungsmittel umfasst.

3. Verfahren nach Anspruch 2, wobei die Lösung auf die Oberfläche des Stents durch eine kapillare Kraft dieser Oberfläche aufgebracht wird.

4. Verfahren nach Anspruch 3, wobei der immunsupprimierende Arzneistoff an der Oberfläche des Stents der kapillaren Kraft folgend ohne Verwendung eines Trägers anhaftet.

5. Verfahren nach Anspruch 4, wobei der immunsupprimierende Arzneistoff in das Netzwerk, die Vertiefungen und die Kapillarien der Mikrostrukturen der Stentoberfläche eindringt.

6. Verfahren nach irgendeinem der vorstehenden Ansprüche, wobei die laterale Abmessungen (d) der Mikrostrukturen im Berrich von 1 bis 3 Mikrometer liegen.

7. Verfahren nach irgendeinem der vorstehenden Ansprüche, wobei die Abstände (e) zwischen benachbarten Mikrostrukturen in dem Bereich von 0.5 bis 5 Mikrometern liegen.

8. Verfahren nach Anspruch 7, wobei die Abstände (e) zwischen benachbarten Mikrostrukturen in dem Bereich von 1 bis 3 Mikrometern liegen.

9. Verfahren nach irgendeinem der vorstehenden Ansprüche, wobei die Oberflächenstruktur des Stents inert ist und keine toxischen Substanzen freisetzt.

10. Verfahren nach irgendeinem der vorstehenden Ansprüche, wobei die Oberflächenstruktur des Stents eine Oberflächenladung in dem Bereich von 3 x 10⁻² bis 5 x 10⁻² N/m (30-50 dynes/cm (g x cm x s⁻²/cm)) aufweist.

11. Verfahren nach irgendeinem der vorstehenden Ansprüche, wobei der immunsupprimierende Arzneistoff in der Lage ist, ein Immunreaktion oder - antwort durch Interaktion mit den T-Zellen des menschlichen Körpers zu unterdrücken.

12. Verfahren nach irgendeinem der vorstehenden Ansprüche, wobei der immunsupprimierende Arzneistoff Sirolimus ist.

13. Verfahren nach irgendeinem der vorstehenden Ansprüche, wobei der immunsupprimierende Arzneistoff Tacrolimus ist.

14. Verfahren nach irgendeinem der vorstehenden Ansprüche, wobei die durch die Mikrostrukturen modifizierte Oberfläche aus Iridiumoxid hergestellt ist.

15. Verfahren nach irgendeinem der vorstehenden Ansprüche, wobei die durch die Mikrostrukturen modifizierte Oberfläche aus Nioboxid hergestellt ist.

16. Verfahren nach irgendeinem der vorstehenden Ansprüche, wobei die durch die Mikrostrukturen modifizierte Oberfläche aus Titannitrat hergestellt ist.

17. Verfahren nach Anspruch 13, wobei das Lösungsmittel Chloroform ist.

18. Verfahren nach Anspruch 17, wobei das Chloroform von der Oberfläche des Stents verdampft wird.

19. Ein Stent zur Implantierung in ein Lumen des menschlichen Körpers, wobei der Stent durch das Verfahren nach irgendeinem der Ansprüche 1 bis 18 herstellbar ist.

## Revendications

1. Procédé de fabrication d'un stent à implanter dans une lumière du corps humain, comprenant les étapes suivantes:
fournir un stent présentant une surface extérieure rugueuse, **caractérisé en ce que** la surface du stent comprend des microstructures présentant des extensions latérales (d) comprises dans la gamme de 0,5 micromètre à 5 micromètres et séparées les unes des autres par des espacements (e), moyennant quoi la reconnaissance du stent en tant que corps étranger implanté est réduite; et
appliquer un médicament immunosuppresseur à la surface extérieure rugueuse, moyennant quoi le médicament immunosuppresseur traite une surface intérieure de la lumière et empêche une réaction immunitaire de celle-ci.

2. Procédé selon la revendication 1, **caractérisé en ce que** l'étape d'application comprend l'immersion du stent dans une solution contenant le médicament immunosuppresseur et un solvant.

3. Procédé selon la revendication 2, **caractérisé en ce que** la solution est appliquée à la surface du stent par l'action d'une force capillaire de ladite surface.

4. Procédé selon la revendication 3, **caractérisé en ce que** le médicament immunosuppresseur adhère à la surface du stent sous l'effet d'une force capillaire sans utiliser de porteur.

5. Procédé selon la revendication 4, **caractérisé en ce que** le médicament immunosuppresseur est imprégné dans le réseau, les rainures et les capillaires des microstructures de la surface du stent.

6. Procédé selon l'une quelconque des revendications précédentes, **caractérisé en ce que** les extensions latérales (d) des microstructures sont comprises dans la gamme de 1 micromètre à 3 micromètres.

7. Procédé selon l'une quelconque des revendications précédentes, **caractérisé en ce que** les espacements (e) entre des microstructures adjacentes sont compris dans la gamme de 0,5 micromètre à 5 micromètres.

8. Procédé selon la revendication 7, **caractérisé en ce que** les espacements (e) entre des microstructures adjacentes sont compris dans la gamme de 1 micromètre à 3 micromètres.

9. Procédé selon l'une quelconque des revendications précédentes, **caractérisé en ce que** la structure de surface du stent est inerte et ne libère aucune substance toxique.

10. Procédé selon l'une quelconque des revendications précédentes, **caractérisé en ce que** la structure de surface du stent présente une charge de surface comprise dans la gamme de 3x10⁻² N/m à 5x10⁻² N/m (30 à 50 dynes/cm (g x cm x s⁻² /cm)).

11. Procédé selon l'une quelconque des revendications précédentes, **caractérisé en ce que** le médicament immunosuppresseur est capable de supprimer une réaction ou une réponse immunitaire qui interagit avec les cellules T du corps humain.

12. Procédé selon l'une quelconque des revendications précédentes, **caractérisé en ce que** le médicament immunosuppresseur est le sirolimus.

13. Procédé selon l'une quelconque des revendications précédentes, **caractérisé en ce que** le médicament immunosuppresseur est le tacrolimus.

14. Procédé selon l'une quelconque des revendications précédentes, **caractérisé en ce que** la surface du stent modifiée par les microstructures est constituée d'oxyde d'iridium.

15. Procédé selon l'une quelconque des revendications précédentes, **caractérisé en ce que** la surface du stent modifiée par les microstructures est constituée d'oxyde de niobium.

16. Procédé selon l'une quelconque des revendications précédentes, **caractérisé en ce que** la surface du stent modifiée par les microstructures est constituée de nitrate de titane.

17. Procédé selon la revendication 13, **caractérisé en ce que** le solvant est le chloroforme.

18. Procédé selon la revendication 17, **caractérisé en outre en ce que** le chloroforme est évaporé à partir de la surface du stent.

19. Stent à implanter dans une lumière du corps humain, ledit stent pouvant être produit en utilisant le procédé selon l'une quelconque des revendications 1 à 18.
